# EUROPEAN PATENT SPECIFICATION

(11) **EP 1 588 723 B1**
(45) Date of publication and mention of the grant of the patent: **03.06.2009**
(21) Application number: 04007523.6
(22) Date of filing: 29.03.2004
(51) Int. Cl.: A61L 15/26, C08L 101/00, A61L 15/18, A61L 15/60

(54) **Absorbent member for absorbent articles comprising swellable polymers of high permeability which are capable of forming hydrogels**
Absorbierendes Element für absorbierende Artikel enthaltend Hydrogel-formende, quellbare Polymere mit hoher Permeabilität
Eléments absorbants destinés à des articles absorbants, comprenant des polymères gonflables ayant une perméabilité élevée et capable de former des hydrogels

(43) Date of publication of application: 26.10.2005
(73) Proprietor: THE PROCTER & GAMBLE COMPANY, Cincinnati, Ohio 45202 (US)
(72) Inventor: Borgmann, Carolin, Michaela, 65760 Eschborn (DE); Lindner, Torsten, 61476 Kronberg (DE)
(74) Representative: Heide, Ute

(56) References cited:
- EP-A- 0 761 241
- EP-A- 0 928 813
- WO-A-02/060983
- US-A- 5 560 929
- US-A1- 2004 048 955

## Description

### Field of the invention

The present invention relates to absorbent members in absorbent cores for absorbent articles, which are intended to receive and retain bodily discharges such as urine. Such articles are disposable hygiene articles like baby diapers, training pants, adult incontinence articles, feminine care articles and the like. The improvement essentially is based on the recognition that replacing most or all of the cushioning fibrous absorbent material in an absorbent core by a liquid storage material capable of retaining liquid while maintaining or improving acquisition behavior is desirable as the reduction in cushioning is more than compensated by the gain in comfort. The comfort however can only be achieved if the more fundamental requirements of a diaper in respect to liquid handling are satisfied or improved. Especially if this liquid handling performance is improved beyond the performance of conventional absorbent structures in order to allow creation of thinner and drier absorbent articles, the users of such articles would experience them as providing a more than expected comfort improvement relative to the thinness gain. To provide such absorbent cores and articles made therewith only became possible with the development of new highly permeable absorbent gel materials especially capable to acquire and conduct liquids at super absorbent polymer concentrations, which are not used today. The second aspect allowing this breakthrough development is the ability to maintain the comfort and performance of such high super absorbent polymer concentration articles during the full usage cycle of the article, from dry to fully loaded, especially by improving the ability of the absorbent members to maintain permeability of such articles during use.

### Background of the invention

Absorbent Articles for receiving and retaining bodily discharges such as urine or faeces such as disposable diapers, training pants, and adult incontinence articles are well known in the art, and significant effort has been spent against improving their performance. Such improvements generally aim at addressing the primary function of such articles, namely retaining body fluids, but also at minimizing the negatives associated with wearing such articles by increasing the comfort of the wearer. One aspect contributing to such comfort is how to pick up and retain the body waste (primarily liquid) in an "absorbent structure", whereby the waste material is acquired by the article, then conducted away from the location of acquisition (distribution, permeation), and then stored (retained).

It is also well established that reducing the thickness of the article by reducing the primary thickness cause, i.e. the absorbent structure helps to improve comfort. This however was always a question of balance between liquid handling performance and thickness. Also a substantial amount of cushioning was considered necessary for comfortable diapers. Finally the skilled person considered it impossible to reduce or even remove the fibrous material to a point where the modem particulate super absorbent materials would take over part or all of the liquid acquisition and distribution functionalities previously provided by fibrous matrixes. Finally even if there were structures which could possibly provide all such beneficial aspects when dry it was completely in-conceivable that this could be build into an absorbent structure such that the liquid handling and comfort performance would be maintained even after the first gushes of liquid had been absorbed.

The development of absorbent structures of particular thinness has also other beneficial aspects making such a development the subject of substantial commercial interest. F or example, thinner diapers are not just less bulky to wear and fit better under clothing they are also more compact in the package, making the diapers easier for the consumer to carry and store. Compactness in packaging also results in reduced distribution costs for the manufacturer and distributor, including less shelf space required in the store per diaper unit.

As indicated the ability to provide thinner absorbent articles such as diapers has been contingent on the ability to develop relatively thin absorbent structures that can acquire and store large quantities of discharged body fluids, in particular urine. In this regard, the use of absorbent polymers often referred to as "hydrogels, "hydrogel-forming polymer "super absorbents" "hydrocolloid", "super absorbent polymer or SAP" has been particularly important. See, for example, the discussion contained in EP 1 393 757 A chapters 8 to 22.

An important property at higher concentrations of these SAPs is their permeability/flow conductivity. Permeability/flow conductivity can be defined in terms of Saline Flow Conductivity (SFC) values. SFC is well established in the art and defined in great detail e.g. in PCT WO-95-26209, page 69 following or EP-B-752892 paragraph 224 following. SFC measures the ability of a material to transport saline fluids, such as the ability of the hydrogel layer formed from the swollen SAP to transport body fluids. Typically, an air-laid web of pulp fibers (e.g., having a density of 0.15 g/cc) will exhibit an SFC value of about 200 x10-7 cm3sec/g. By contrast, typical SAPs such as Aqualic L-74 (made by Nippon Shokubai Co., LTD) and Nalco-1180 (made by Nalco Chemical Co.) exhibit SFC values of at most 1 x10-7 cm3sec/g. Accordingly, it would be highly desirable to be able to use SAPs that more closely approach an air-laid web of wood pulp fibers in terms of SFC.

Accordingly, it would be desirable to be able to provide an absorbent member comprising: (1) a region or regions having a relatively high concentration of SAP particles; (2) with relatively high permeability/flow conductivity properties more like an air-laid fibrous web; (3) that can readily acquire fluids from or even provide the function of high capillary suction acquisition layers under typical usage conditions / pressures even when subjected to normal use forces. It would also be highly desirable to be able to use SAPs in these absorbent members that, when swollen by body fluids, continue to have a good permeability, improved wicking and/or improved swelling properties.

### SUMMARY OF THE INVENTION

The present invention and its characteristics are defined in the independent claims and preferred embodiments are specified in the dependant claims. In particular the present invention relates to absorbent cores useful in the provision of absorbent incontinence articles such as baby diapers or adult incontinence articles, which articles preferably comprise a topsheet with the absorbent core positioned (immediately) adjacent to it and further optionally a backsheet which together with the topsheet sandwiches the absorbent core.

The absorbent cores of the present invention are especially useful for collection of bodily liquids such as urine. The absorbent core is a component of an absorbent article that is primarily responsible for the fluid handling properties of the article, including acquiring, transporting, distributing and storing body fluids. As such, the absorbent core typically does not include the topsheet or backsheet of the absorbent article. The absorbent core can be provided by a number of absorbent members. An absorbent member refers to a component of the absorbent core that typically provides one or more fluid handling properties, e.g., fluid acquisition, fluid distribution, fluid transportation, fluid storage, etc. An absorbent member can provide the entire absorbent core or only a portion of the absorbent core, i.e., the absorbent core can comprise one or more absorbent members.

According to the present invention an absorbent member, comprised in an absorbent core, is provided with a swellable hydrogel-forming polymer. This swellable hydrogel-forming polymer comprises in combination at least one hydrophilic polymer with dendritic structure and at least one water-insoluble phosphate.

In a preferred embodiment the hydrophilic polymer with dendritic structure is a polyester made of a polyol and 2,2-dimethylolpropione acid, alternatively the hydrophilic polymer can be selected from the group of polypropylenimine, polyamidoamine, polyesteramide or combinations thereof.

In preferred embodiments the water-insoluble phosphate is a calcium phosphate. In other preferred alternatives or in combination with the previous embodiment the hydrogel-forming polymer can comprise a powdered and/or dusty additive, preferably the additive is selected from the group of a metallic salt, a pyrogenic silicic acid, a polysaccharide, a non-ionic tenside, a wax, diatomaceous earth or combination thereof.

The hydrogel forming polymer according to the present invention preferably has a high saline flow conductivity, preferably a saline flow conductivity of at least 80 x 10⁻⁷ cm³ s g ⁻¹, preferably at least 120 x 10⁻⁷ cm³ s g ⁻¹.

In particularly beneficial embodiments of the absorbent core according to the present invention the absorbent member comprises at least 50%, preferably at least 60%, more preferably at least 75%, and most preferably at least 85%, by weight of the hydrogel-forming polymer. When the absorbent core is imployed in an absorbent article comprising a liquid pervious topsheet for receiving body liquids such as urine, the core is preferably positioned adjacent to the topsheet such that the absorbent member is located as close as possible to the topsheet.

### DETAILED DESCRIPTION OF THE INVENTION.

### A. Definitions

As used herein, the term "absorbent core" refers to a component of an absorbent article that is primarily responsible for fluid handling properties of the article, including acquiring, transporting, distributing and storing body fluids. As such, the absorbent core typically does not include the topsheet or backsheet of the absorbent article.

As used herein, the term "absorbent member" refers to the components of the absorbent core that typically provide one or more fluid handling properties, e.g., fluid acquisition, fluid distribution, fluid transportation, fluid storage, etc. The absorbent member can provide the entire absorbent core or only a portion of the absorbent core, i.e., the absorbent core can comprise one or more absorbent members.

As used herein, the term "comprising" means that e.g. various components, members, steps and the like can be conjointly employed according to the present invention. Accordingly, the term "comprising" encompasses the more restrictive terms "made of" and "consisting of," these latter, more restrictive terms having their standard meaning as understood in the art.

All percentages, ratios and proportions used herein are by weight unless otherwise specified.

### B. Material and Components of the Absorbent Core

### a. Chemical Composition of hydrogel forming absorbent polymers

The SAP, super absorbent polymers (SAPs) or hydrogel-forming polymers, useful in the present invention include a variety of water-insoluble, but water-swellable polymers capable of absorbing large quantities of fluids. Such polymer materials are generally known in the art and include all those well-known polymers used or deemed useful in the context of disposable absorbent article technology. Particularly the SAPs disclosed in EP-A-752 892 or those disclosed in a textbook entitled "Modem Super Absorbent Technology" by F.L. Buchholz and A.T. Graham, published by Wiley VCH, New York, 1998 are useful in the context of the present invention.

Preferred polymer materials for use in making SAPs are slightly network cross linked polymers of partially neutralized polyacrylic acids and starch derivatives thereof. Preferably, the SAPs comprise from about 50 to about 95%, preferably about 75%, neutralized, slightly network cross linked, polyacrylic a cid (i.e., poly (sodium acrylate/acrylic acid)). Network cross-linking renders the polymer substantially water-insoluble and, in part, determines the absorptive capacity and extractable polymer content characteristics of the hydrogel-forming absorbent polymers. Processes for network cross linking these polymers and typical network cross-linking agents are described in greater detail in U.S. Patent 4,076,663 or references cited supra.

While the SAP is preferably of one type (i.e., homogeneous), mixtures of polymers can also be used in the present invention. The SAPs useful in the present invention can have a size, shape, and/or morphology varying over a wide range. According to a preferred embodiment of the present invention these polymers are in the form of particles that do not have a large ratio of greatest dimension to smallest dimension (e.g., granules, pulverulents, inter-particle aggregates, inter-particle cross linked aggregates, and the like). The SAPs can also comprise mixtures with low levels of one or more additives, such as for example powdered silica, surfactants, glue, binders, and the like.

For particles as described above, particle size is defined as the dimension determined by sieve size analysis. The methods for determining particle sizes of the SAP particles are described in U.S. Patent 5,061,259 (Goldman et. al), issued October 29, 1991.

Surprisingly it was now found that including hydrophilic polymers with aborescent, especially dendritic structure in the production of SAPs is useful to provide superabsorbers with a small abrasion dust content, in particular after mechanical stressing, with an improved ability of binding powdered and/or dust-like additives, with a high swelling speed, with a high rate of internal liquid transfer and with optimal flow characteristics

According to Römpp's Encyclopedia of Chemistry, published by Georg Thieme Verlag in Stuttgart Germany, 10th edition, page 898 (Römpp, Lexikon-Chemie, Georg Thieme Verlag, Stuttgart, 10. Auflage, Seite 898), dendritic polymers are synthetic macromolecules constructed by step-for-step linkage of two or more monomers in each case with every already bound monomer, with the result that the number of monomer end-groups increases exponentially with every step and ultimately a spherical tree structure is created.

The hydrophilic polymers with dendritic structure, which can be used according to the present invention are polyols with at least 8, preferably at least 16 and most preferably 32 hydroxyl groups as well as a non linear, preferably at least 14-fold and most preferably 30-fold branched fundamental structure.

Hydrophilic polymers with dendritic structures are for example polyesters obtained from a starting polyol by esterification with a C₃-C₂₀ hydroxycarboxylic acid, preferably with a C₄-C₁₂ hydroxycarboxylic acid and most preferably with a C₄-C₈ hydroxycarboxylic acid wherein the hydroxycarboxylic acid exhibits at least two hydroxyl groups, preferably two hydroxyl groups and/or at least two carboxylic acid groups. Of particular preference are hydroxycarboxylic acids with two hydroxyl groups and one carboxylic acid group, in particular 2,2-dimethylol propionic acid. Polyols are compounds with at least two hydroxyl groups, such as ethylene glycol, diethylene glycol, triethylene glycol, polyethylene glycol, propylene glycol, dipropylene glycol, tripropylene glycol, polypropylene glycol, butylene glycol, 1,3-propanediol, 1,4-butanediol, bisphenol A, glycerine, trimethylol propane, pentaerythritol and/or sorbitol. Preferred are dendritic polyesters, of particular preference are Boltorn® 20, Boltorn® 30, Boltorn® 40 und Boltorn® 310 (Perstorp Specialty Chemicals AB, SE).

Hydrophilic polymers with dendritic structure also include polymers obtainable by condensation of polyols with at least two hydroxyl groups and subsequent alkoxylation. Examples in this case are branched polyethylene glycols obtainable via condensation of glycerin molecules and subsequent ethoxylation.

In addition to the above, the hydrophilic polymers with dendritic structure also include all polymers obtainable through polymerization of a monomer with at least one hydroxyl group and subsequent alkoxylation. Polymerization is preferably carried out in the presence of a cross-linking agent. Polymer particles which are hydrophilic on their surface and exhibit numerous hydroxyl groups can be obtained. So-called star-shaped polyethylene glycols, obtained by radical polymerization of p-hydroxyethylstyrene and subsequent alkoxylation, are as examples of the above in Makromol. Chem. 189, 2885 (1988).

Further examples of polymers used according to the invention are highly branched polymers of trade mark HYBRANE® as well as Astramol-Dendrimere® (DSM N.V., NL). Included in this category are particularly highly branched poly(propylene imines), starting, for example, with butylene diamine and obtained via repeated multiple Michael condensation with acrylonitrile and hydrogenation, star-polycaprolactons, star-nylon-6, highly branched polyester amides, based, for example, on the addition product of succinic anhydride and diethanolamine in 1:1 molar ratio. In the process according to the invention, so-called PAMAM Dendrimere based on poly(amidoamine), starting for example, with ammonia and obtained via repeated multiple conversion with methyl acrylate and ethylene diamine can also be used.

Preferred in accordance with the present invention are hydrophilic polymers with dendritic structure and a glass transition temperature T_{g} between 20 and 200°C, particularly preferable between 25 and 50°C and/or an average molecular weight between 1000 and 10000 g/mol, most preferably between 2000 and 6000 g/mol.

According to the invention, 0.005 to 10 weight percent of the hydrophilic polymers are preferred. Better yet between 0.01 and 5 weight percent and more preferred between 0,05 and 1 weight percent, most preferred between 0.10 to 0.80 weight percent of hydrophilic polymer with dendritic structure are employed, with the percentages refering the swellable hydrogel-forming polymer.

Hydrophilic polymers with dendritic structure are preferably mixed with the dried water-absorbing hydrogel. Dry preferably means less than 20% by weight of water content, particularly preferred are less than 10% by weight of water content. The hydrophilic polymer with dendritic structure may, however, be added to the swellable hydrogel-forming polymer prior to, during or after the surface cross-linking process. Preferably, however, addition or/and coating takes place during a surface cross-linking process. The method of intermixing is subject to no limitations.

Where the coating is accompanied by surface cross-linking of the base hydrogel-forming polymer, the surface cross-linking agent may be brought into solution together with the dendritic polymer, or alternatively by separate addition into the surface cross-linking mixer. Where dust-like or powdered additive are also coated onto the base polymer, the possibility exists that the dendritic polymer is dissolved in a solvent wherein the dust-like or powdered additive is also dispersed. As an option, this mixture may also contain the surface cross-linking agent. Suitable solvents are all those known to any person skilled in the art which may be used for surface cross-linking, preferably water.

In a preferred embodiment according to the invention the swellabel hydrogel-forming polymers comprise a fraction of particles of diameter less than 10 µm and less than 100 ppm by weight, preferably less than 50 ppm by weight and more preferably less than 10 ppm by weight wherein the swellable hydrogel-forming polymer comprises at least one powdered or dust-like additive such as metal salts, aluminum sulfate and/or magnesium sulfate, pyrogenic silicic acids such as Aerosil® 200, polysaccharides and their derivatives, non-ionic surfactants, waxes, diatom earths and/or microscopic hollow inclusions.

In accordance with the present invention the hydrogel-forming polymers also comprise water insoluble phosphates such as calcium phosphate. The term "Water insoluble" signifies a solubility of less than 1 g, preferably less than 0.1 g and more preferably less than 0.01 g in 100 ml of water at 25°C.

Microscopic hollow inclusions are described in Chem. Ing. Techn. 75, 669 (2003). Microscopic hollow inclusions are gas-filled or evacuated globular solid-state particles with diameters between 1 and 1000 µm. Typical wall-thicknesses of the microscopic hollow inclusions are between 1 and 10% of their diameter. The wall materials are subject to no limitations. Possible wall materials are glass, ceramic-forming oxides or mixed oxides, silicates, alumosilicates, polymers, polycondensates and metals.

Swellable hydrogel-forming polymers according to the invention typically have a Saline flow conductivity (SFC) of at least 40 x 10⁻⁷ cm³s/g, preferably of at least 60 x 10⁻⁷ cm³s/g, of particular preference 80 x 10⁻⁷ cm³s/g, even more preferably of at least 120 x 10⁻⁷ cm³s/g and most preferably of at least 130 x 10⁻⁷ cm³s/g.

Swellable hydrogel-forming polymers according to the invention typically have a centrifugal retention capacity (CRC) of at least 24 g/g, preferably at least 25 g/g, more preferably at least 26 g/g, even more preferably at least 28 g/g and most preferably at least 30 g/g.

Powdered additives preferably have an average particle size of less than 200 µm, of particular preference less than 400 µm. Dust-like additives preferably have an average particle size of less than 200 µm, preferably of less than 50 µm and of particular preference less than 10 µm.

### b. Physical Properties

### (1). Saline Flow Conductivity (SFC)

An important characteristic and mandatory for the SAPs useful in the present invention is their permeability or flow conductivity when swollen with body fluids so as to form a hydrogel zone or layer. This permeability or flow conductivity is defined herein in terms of the Saline Flow Conductivity (SFC) value of the SAP. SFC measures the ability of a formed hydrogel layer to transport or distribute body fluids under usage pressures. The method for determining a SFC value is provided e.g. in EP-B-752 892 paragraph 224 following. The SFC value of the SAPs useful in the present invention has already been mentioned in terms of units. As expressed herein the numerical value of a unit is 10-7 cm³sec/g (or cm³sec/g/10,000,000). In other words 40 SFC units means an SFU value of 40 times 10-7 cm3sec/g.

### (2). Centrifuge Retention Capacity (CRC)

For most hydrogel-forming absorbent polymers, gel volume as a measurement of absorbent capacity is determined by the method described in U.S. Reissue Patent 32,649 (Brandt et al), reissued April 19, 1988 but using 0.9% saline solution instead of synthetic urine. The gel volume as well as the CRC capacity is calculated on a dry-weight basis. An alternative method for measuring gel volume can be used for SAPs that adsorb Blue Dextran (see gel volume method in Re 32,649) to the surfaces of the formed hydrogel (e.g., polymers prepared from cationic monomers). For these hydrogel-forming polymers, the Absorptive Capacity test is used, but the dry weight of the hydrogel-forming polymer is used in the calculation instead of the as-is weight. See e.g. U.S. Patent 5,124,188 (Roe et al), issued June 23, 1992 at Columns 27-28 for description of the Absorptive Capacity test.

For the evaluation of the centrifuge retention capacity it has been found that the so-called tea-bag-evaluation or measurement (hereinafter CRC measurement) is most appropriate to reflect the maintenance of capillary pressure at situations approaching saturation of the absorbent capability of a SAP material. For the test standard laboratory conditions (21-23 °C, 50% relative humidity) are used. Sample SAP material is kept dry in a tightly closing flask or other container, which is only opened upon start of the evaluation. Other material used in the evaluation (tissues, equipment etc.) is conditioned for 24 hours prior to measurements at the above laboratory conditions.

For the CRC measurement 0.2 +/- 0.0050 g of SAP particles are put into a tea bag (the bag needs to be freely liquid pervious and must retain the particles, i.e. the tea bag pores need to be not larger than the smallest particles. The tea bag should have a size of 60mm x 85mm and is sealed by welding after filling. The tea bag is then immersed for 30 minutes in a 0.9% saline solution such that there is at least 0.83 1 of solution per gram of SAP; preferably there is a substantial excess of this ratio. After the 30 minute immersion the tea bag is centrifuged at 250 g for 3 minutes to remove excess saline solution. The bag is weight to the nearest 0.01 g and the absorbed liquid is calculated. The result is reported by using the amount of dry SAP, which was put into the tea bag, as grams absorbed per gram of SAP particles.

### c. Absorbent Cores containing SAPs

According to the present invention absorbent members as part of absorbent cores for disposable absorbent articles comprise the previously described SAPs, with or without other optional components such as fibers, distribution / acquisition or thermoplastic material, foams, scrims etc. These absorbent members function as fluid storage members, even when subjected to pressures and tensions and torsions normally encountered as a result of the wearer's movements of absorbent articles made therewith. It should be understood, however, that such polymer-containing absorbent members can also serve several functions integrated in a single member.

An important aspect of these absorbent members according to the present invention is that they contain one or more regions having a high concentration of these SAPs in order to provide relatively thin absorbent articles capable of acquiring, distributing, absorbing and retaining large quantities of body fluids. A high concentration of SAPs, in accordance with the present invention, is desirable to reduce the level of other components, in particular fibrous components.

In measuring the concentration of SAP an absorbent member or in a given region of an absorbent member, the percent by weight of the SAP relative to the combined weight of SAP and any other components (e.g., fibers, thermoplastic material, etc.) that are present in the member, respectively in the same region containing the polymer is used. With this in mind, the concentration of the SAPs in a given region of an absorbent member according to the present invention can be in the range of from about 50 to 100%, preferably from about 60 to 100%, more preferably from about 75 to 100%, and most preferably from about 85% to 100%.

Absorbent members according to the present invention comprising high concentrations of SAPs are useful alone or in combination with other absorbent members as part of the absorbent cores and articles according to the present invention. Depending on the intended use, the preferred cores according the present invention comprise the SAPs according to the present invention in a basis weight of at least 20 g/m², preferably at least 100 g/m² and even more preferably of at least 150 g/m².

The preferred absorbent members in the cores according to the present invention can include those useful for initially acquiring the discharged body fluids and distributing these fluids to the fluid storage member. These preferred absorbent members can provide multiple fluid handling properties (e.g., fluid acquisition and distribution) or single fluid handling properties (e.g., fluid distribution). Other absorbent members can also comprise lower concentrations of the SAPs that have the physical properties previously specified or can comprise SAPs having different physical properties. The preferred absorbent members are preferably located close, more preferably immediately adjacent , to the topsheet, which is exposed to the source of the liquid to be absorbed.

### d. Absorbent Articles

Because of the unique absorbent properties of the absorbent cores of the present invention, they are especially suitable for use in disposable absorbent articles for absorption of urine (also referred to as disposable absorbent incontinence articles). These absorbent articles typically comprise a liquid impervious (but preferably gas pervious) backsheet, a fluid pervious topsheet joined to, or otherwise associated with the backsheet, and the absorbent core according to the present invention positioned between the backsheet and the topsheet. Such articles are well known in the art and fully disclosed in various documents mentioned throughout the description e.g. in EP 752 892.

Parallel with the present specification a detailed description of the making and evaluating processes used for making SAP particles according to the present invention is filed in a patent application entitled "Absorbent member for absorbent articles comprising swellable polymers of high permeability which are capable of forming hydrogels" by applicant/assignee BASF, Ludwigshafen of Germany. A copy of this application is available in the file of the European patent office of the present application.

## Claims

1. Absorbent core for collection of body liquids such as urine said core comprising an absorbent member, said member comprising a swellable hydrogel-forming polymer comprising in combination
at least one hydrophilic polymer with dendritic structure and
at least one water-insoluble phosphate.

2. An absorbent core according to Claim 1 **characterized in that** the hydrophilic polymer with dendritic structure is a polyester made of a polyol and 2,2-dimethylolpropione acid.

3. An absorbent core according to Claim 1 **characterized in that** the hydrophilic polymer with dendritic structure is selected from the group of polypropylenimine, a polyamidoamine or a polyesteramide or combinations thereof.

4. An absorbent core according to Claims 1 to 3 **characterized in that** the water-insoluble phosphate is calcium phosphate.

5. An absorbent core according to Claims 1 to 4 **characterized in that** the hydrogel forming polymer comprises a powdered and/or dusty additive.

6. An absorbent core according to Claim 5 **characterized in that** said additive is selected from the group of a metallic salt, a pyrogenic silicic acid, a polysaccharide, a non-ionic tenside, a wax, diatomaceous earth or combination thereof.

7. An absorbent core according to any of any of the preceding claims, **characterized in that** said hydrogel forming polymer has a saline flow conductivity of at least 80 × 10⁻⁷ cm³ s g⁻¹, preferably at least 120 × 10⁻⁷ cm³ s g⁻¹.

8. An absorbent core according to any of the preceding claims **characterized in that** said absorbent member comprises at least 50%, preferably at least 60%, more preferably at least 75%, and most preferably at least 85%, by weight of said hydrogel-forming polymer.

9. An absorbent article comprising a liquid pervious topsheet for receiving body liquids such as urine, said article comprising further an absorbent core according to any of the preceding claims **characterized in that** said core is positioned adjacent to said topsheet such that said absorbent member is located as close as possible to said topsheet.

## Patentansprüche

1. Absorptionskern zum Auffangen von Körperflüssigkeiten wie Urin, wobei der Kern ein Absorptionselement umfasst, wobei das Element ein quellbares hydrogelbildendes Polymer umfasst, das in Kombination Folgendes umfasst:
mindestens ein hydrophiles Polymer mit dendritischer Struktur und
mindestens ein wasserunlösliches Phosphat.

2. Absorptionskern nach Anspruch 1, **dadurch gekennzeichnet, dass** das hydrophile Polymer mit dendritischer Struktur ein Polyester aus einem Polyol und 2,2-Dimethylolpropionsäure ist.

3. Absorptionskern nach Anspruch 1, **dadurch gekennzeichnet, dass** das hydrophile Polymer mit dendritischer Struktur ausgewählt ist aus der Gruppe von Polypropylenimin, einem Polyamidoamin oder einem Polyesteramid oder Kombinationen davon.

4. Absorptionskern nach den Ansprüchen 1 bis 3, **dadurch gekennzeichnet, dass** das wasserunlösliche Phosphat Calciumphosphat ist.

5. Absorptionskern nach den Ansprüchen 1 bis 4, **dadurch gekennzeichnet, dass** das hydrogelbildende Polymer einen pulverförmigen und/oder staubförmigen Zusatzstoff umfasst.

6. Absorptionskern nach Anspruch 5, **dadurch gekennzeichnet, dass** der Zusatzstoff ausgewählt ist aus der Gruppe von einem Metallsalz, einer pyrogenen Kieselsäure, einem Polysaccharid, einem nichtionischen Tensid, einem Wachs, einer Diatomeerde oder Kombinationen davon.

7. Absorptionskern nach einem der vorstehenden Ansprüche, **dadurch gekennzeichnet, dass** das hydrogelbildende Polymer eine Flüssigkeitsleitfähigkeit von mindestens 80 × 10⁻⁷ cm³sg⁻¹, vorzugsweise mindestens 120 × 10⁻⁷ cm³sg⁻¹ aufweist.

8. Absorptionskern nach einem der vorstehenden Ansprüche, **dadurch gekennzeichnet, dass** das Absorptionselement mindestens 50 Gew.-%, vorzugsweise mindestens 60 Gew.%, mehr bevorzugt mindestens 75 Gew.% und am meisten bevorzugt mindestens 85 Gew.-% des hydrogelbildenden Polymers umfasst.

9. Absorptionsartikel, der eine flüssigkeitsdurchlässige Oberschicht zum Auffangen von Körperflüssigkeiten wie Urin umfasst, wobei der Artikel ferner einen Absorptionskern nach einem der vorstehenden Ansprüche umfasst, **dadurch gekennzeichnet, dass** der Kern angrenzend an die Oberschicht angeordnet ist, so dass das Absorptionselement so nahe wie möglich an der Oberschicht angeordnet ist.

## Revendications

1. Âme absorbante pour un recueil de liquides corporels tels que l'urine, ladite âme comprenant un élément absorbant, ledit élément comprenant un polymère formant hydrogel pouvant gonfler comprenant en combinaison
au moins un polymère hydrophile avec une structure dendritique et
au moins un phosphate insoluble dans l'eau.

2. Âme absorbante selon la revendication 1, **caractérisée en ce que** le polymère hydrophile avec une structure dendritique est un polyester constitué d'un polyol et d'un acide 2,2-diméthylolpropione.

3. Âme absorbante selon la revendication 1, **caractérisée en ce que** le polymère hydrophile avec une structure dendritique est choisi dans le groupe d'une polypropylène-imine, une polyamido-amine ou un polyesteramide ou leurs combinaisons.

4. Âme absorbante selon les revendications 1 à 3, **caractérisée en ce que** le phosphate insoluble dans l'eau est du phosphate de calcium.

5. Âme absorbante selon les revendications 1 à 4, **caractérisée en ce que** le polymère formant hydrogel comprend un additif pulvérulent et/ou poussiéreux.

6. Âme absorbante selon la revendication 5, **caractérisée en ce que** ledit additif est choisi dans le groupe d'un sel métallique, un acide silicique pyrogène, un polysaccharide, un tensioactif non ionique, une cire, une terre à diatomées ou une de leur combinaison.

7. Âme absorbante selon l'une quelconque des revendications précédentes, **caractérisée en ce que** ledit polymère formant hydrogel a une conductivité en flux salin d'au moins 80 × 10⁻⁷ cm³sg⁻¹, de préférence au moins 120 × 10⁻⁷ cm³sg⁻¹.

8. Âme absorbante selon l'une quelconque des revendications précédentes, **caractérisée en ce que** ledit élément absorbant comprend au moins 50 %, de préférence au moins 60 %, plus préférablement au moins 75 %, et le plus préférablement au moins 85 % en poids dudit polymère formant hydrogel.

9. Article absorbant comprenant une feuille de dessus perméable aux liquides pour recevoir des liquides corporels tels que de l'urine, ledit article comprenant en outre une âme absorbante selon l'une quelconque des revendications précédentes, **caractérisé en ce que** ladite âme est positionnée adjacente à ladite feuille de dessus de telle sorte que ledit élément absorbant est situé le plus près possible de ladite feuille de dessus.
